(19) Europäisches Patentamt
European Patent Office
Office européen des brevets

(11) **EP 4 656 182 B1**

(12) **EUROPEAN PATENT SPECIFICATION**

(45) Date of publication and mention
of the grant of the patent:
**18.03.2026   Bulletin 2026/12**

(21) Application number: **25155776.5**

(22) Date of filing: **04.02.2025**

(51) International Patent Classification (IPC):
*A61K 9/00* (2006.01)      *A61K 9/08* (2006.01)
*A61K 9/19* (2006.01)      *A61K 38/26* (2006.01)
*A61K 47/02* (2006.01)      *A61K 47/18* (2017.01)
*A61K 47/26* (2006.01)

(52) Cooperative Patent Classification (CPC):
**A61K 9/0019; A61K 9/08; A61K 38/26;**
A61K 47/02; A61K 47/183; A61K 47/26

(54) **GLP-1 ANALOGUE COMPOSITIONS AND PREPARATION METHOD**

GLP-1-ANALOGZUSAMMENSETZUNGEN UND HERSTELLUNGSVERFAHREN

COMPOSITIONS D'ANALOGUES DE GLP-1 ET PROCÉDÉ DE PRÉPARATION

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **31.05.2024  EP 24382587**

(43) Date of publication of application:
**03.12.2025   Bulletin 2025/49**

(73) Proprietor: **Galenicum Health S.L.U.
08950 Esplugues de Llobregat (ES)**

(72) Inventors:
• **Gómez Coello, Luis
Esplugues de Llobregat (ES)**
• **Osuna López, Jaime
Esplugues de Llobregat (ES)**

(74) Representative: **Galenicum Health S.L.U.
CL Sant Gabriel n°50
08950 Esplugues de Llobregat (ES)**

(56) References cited:
WO-A1-2020/127476      WO-A1-2021/123228
US-A1- 2023 159 606      US-B2- 8 748 376

**Description**

The present invention relates to methods for the preparation of a pharmaceutical composition comprising a GLP-1 agonist. The invention also relates to the pharmaceutical composition prepared according to the said methods and to cartridges comprising the said pharmaceutical composition.

**BACKGROUND**

The development of pharmaceutical compositions comprising therapeutic peptides such as glucagon-like peptide-1 (GLP-1) analogues or agonists is of significant interest due to their potential in treating conditions like diabetes and obesity. GLP-1 analogues are susceptible to degradation when formulated as pharmaceuticals, and the process of preparing these compositions requires careful consideration of factors such as stability, efficacy, and safety of the product. However, these peptides are prone to chemical instability, such as oxidation or hydrolysis, which may affect their structural integrity and therapeutic function, and also to physical instability, such as aggregation or precipitation, which may lead to reduced bioavailability and possible immunogenic reactions. The combination of these factors makes the development of stable GLP-1 analogue formulations a complex and critical task in pharmaceutical sciences.

Particularly, fibril formation, a process by which a GLP-1 analogues tends to form well-ordered, thread-like macromolecular aggregates, poses a significant challenge in a GLP-1 analogue formulation. In the literature, heat treatment of GLP-1 analogue solutions has been suggested for increasing the shelf life and stability of the pharmaceutical solutions due to fibril formation.

Fibrils in parenteral compositions present several significant disadvantages that impact the safety, efficacy, and regulatory compliance of pharmaceutical products. Immunogenicity is a major concern, as fibrils can provoke an immune response, potentially leading to adverse reactions in patients, ranging from mild allergic responses to severe immunological complications. The presence of fibrils also signifies a loss of therapeutic efficacy, as aggregated proteins are often denatured and incapable of performing their intended biological functions. This aggregation indicates instability, compromising the product's shelf-life and necessitating stringent storage conditions.

Additionally, the physical presence of fibrils can pose safety risks, such as causing blockages in blood vessels or tissues, which could lead to serious health issues like embolism or inflammation. Hence, minimizing fibril formation is crucial for ensuring the overall integrity and success of parenteral pharmaceutical applications.

US 2023/159606 A1 discloses methods to prepare GLP-1 agonist products with low amounts of aggregates by optimizing the pH conditions and the selection of excipients and by depleting dissolved oxygen during manufacturing.

According to WO 2006051110 A2 and US 8 748 376 B2, fibrillation in GLP-1 analogue solutions can be reduced by heating the solution of said GLP-1 analogues between 50°C and 95°C, at a pH between 8.0 to 10.5 and then continue the heating for between 3 minutes and 180 minutes. This method would allow the fibrils to dissolve in their initial state and delay its formation.

WO 2020127476 A1 and WO 2021123228 A1 disclose methods for the preparation of pharmaceutical solution comprising a GLP-1 analogue which involves heating the solution to a temperature of 26-49°C.

However, due to the importance of therapeutic peptides, there is a continuous need for innovative approaches that can enhance the stability of GLP-1 analogue formulations, thereby improving their therapeutic effectiveness and enhancing their stability in the pharmaceutical product lifecycle.

The present invention is directed towards addressing the shortcomings and challenges outlined above.

**SUMMARY OF THE INVENTION**

The present invention provides a method for the preparation of a pharmaceutical composition comprising a GLP-1 agonist having an increased stability and less tendency to fibrils formation.

Heat treatment of peptide solutions has been described in the literature for providing stable pharmaceutical solutions. However, the inventors have surprisingly found that stable pharmaceutical formulations comprising a GLP-1 agonist can be prepared without involving thermal treatment of the solutions. It has been observed that GLP-1 agonist compositions prepared according to the invention have a reduced number of fibrils and content of impurities, avoiding its formation in the manufacturing process.

In addition, the present invention aims to provide safe and stable pharmaceutical compositions that are cost-effective and straightforward to manufacture at industrial scale.

Each of the aspects and embodiments of the present invention described herein may be combined with one or more aspects and embodiments of the present invention.

In a first aspect, the invention concerns a method for the preparation of a pharmaceutical composition comprising a GLP-1 agonist, a tonicity modifier, a buffering agent and a preservative which comprises the following steps:

a. preparing a final solution comprising the tonicity modifier, the buffering agent, the preservative, the GLP-1 agonist and water for injection; and

b. adjusting the pH of the final solution between 6.5-8.5, preferably between 7.5-8.5, with a pH adjuster;

wherein the final solution is prepared at a temperature between 2-15°C, preferably 2-10°C, more preferably 2-8 °C, most preferably 3-6°C.

The pharmaceutical solutions prepared according to a first aspect of the invention have lower number of fibrils, i.e., less fibrillation over time and less individual and total impurities than pharmaceutical solutions which had undergone heat treatment.

In a further disclosure, a pharmaceutical composition comprising a GLP-1 agonist, a tonicity modifier, a buffering agent and a preservative wherein the fibrils concentration in the pharmaceutical composition is less than 0.02 $\mu$M as measured by Thioflavin T (ThT) Fibrillation Estimation Assay is disclosed.

## DETAILED DESCRIPTION OF THE INVENTION

As used in the present disclosure, the following words, phrases, and symbols are generally intended to have the meanings as set forth below, except to the extent that the context in which they are used indicates otherwise.

All percentages are expressed by weight (w/w) and as used herein are referred to the total weight of the composition, unless specifically noted otherwise.

The term "active ingredient" as used herein refers to a therapeutically active compound, as well as any pharmaceutically acceptable hydrates and solvates of the compound.

The term "buffer" as used herein refers to a chemical compound in a pharmaceutical composition that reduces the tendency of pH of the composition to change over time as would otherwise occur due to chemical reactions. Examples of buffers include sodium dihydrogen phosphate, dibasic sodium phosphate, phosphoric acid, sodium phosphate, acetic acid, sodium acetate, carbonic acid, sodium carbonate, citric acid, citrate, meglumine, glycine, histidine, lysine, arginine, asparagine, glutamic acid, sodium glutamate, tris (hydroxymethyl)-aminomethane, methionine, Hepes, maleic acid, malic acid, lactate, etc.

The term "pH adjuster" as used herein refers to pharmaceutically acceptable excipients which are added to the solution of the active ingredient to adjust the pH to a certain value. Such pH adjusters can be alkaline or acid agents and may comprise inorganic salts as well as organic acids or salts of organic acids. Examples of preferred pH adjusters are HCl and/or NaOH.

The term "pharmaceutically acceptable" as used herein indicates that the substance or composition must be compatible chemically and/or toxicologically, with the other ingredients in the composition, and/or the mammal (e.g. human) being treated therewith.

The term "pharmaceutical composition" as used herein means a product comprising an active ingredient or a salt thereof together with pharmaceutical excipients such as buffer, preservative and tonicity modifier, said pharmaceutical composition being useful for treating, preventing or reducing the severity of a disease or disorder by administration of said pharmaceutical composition to a person. The expression "pharmaceutical composition" may also be referred to as a "pharmaceutical formulation".

The term "preservative" as used herein refers to a chemical compound which is added to a pharmaceutical composition to prevent or delay microbial activity (growth and metabolism). Examples of pharmaceutically acceptable preservatives are phenol, m-cresol, methyl p-hydroxybenzoate, propyl p-hydroxybenzoate, benzoic acid, benzyl alcohol, benzyl benzoate, 2-phenoxyethanol, butyl p-hydroxybenzoate, 2-phenylethanol, benzyl alcohol, chlorobutanol, acetone sodium bisulfite, benzalkonium chloride, benzethonium chloride and thiomerosal.

The term "stable" as used herein refers to any pharmaceutical composition comprising the active ingredient having a sufficient physical and chemical stability to allow storage under any of the general storage conditions as defined by ICH Q1A (R2).

The term "tonicity modifier" as used refers to a chemical compound in a pharmaceutical composition that serves to modify the osmotic pressure of the pharmaceutical composition so that the osmotic pressure becomes closer to that of human plasma. The tonicity modifier is also known in the art as "isotonicity agent". Isotonicity agents include mannitol, sorbitol, lactose, propylene glycol dextrose, trehalose, sodium chloride, potassium chloride, glycerol, glycerin, etc.

The quantitative determination of liraglutide by HPLC analysis was carried out using ACQUITY UPLC H-Class PLUS system, Aeris Peptide XB C18 100Å 4.6 mm x 250 mm, 3.6 $\mu$m column was used. A Security Guard ULTRA cartridge, UHPLC C18-Peptide 4.6 mm ID pre-column was used. The apparatus was equipped with a manual injector and UV detector. The injection valve was a Rheodyne with a capacity of 20 $\mu$L. Mobile phase A (0.5% TFA in Water: Methanol (95:5, v/v%)) and Mobile phase B (0.5% TFA in Acetonitrile:Methanol: Water (90:5:5, v/v/v%) with 6.0 mL NH3 30%) were used at a ratio 40:60. As a diluent, 0,025% v/v Ammonia in water was used. The mobile phases were filtered through a 0.45 $\mu$m membrane filter and sonicated before use. It was pumped through the column at a flow rate of 0.8 mL/min. Injection

volume was 10 $\mu$L and the column was maintained at 25°C. The detection was monitored at 220 nm and the run time was set as 35 minutes. The amount of liraglutide in the samples was determined by comparison with appropriate external standard curves obtained applying the least square linear regression analysis.

The quantitative determination of liraglutide impurities by UPLC was carried out using an ACQUITY UPLC H-Class PLUS system, Acquity UPLC Peptide CSH C18 130Å 2.1 x 150mm, 1.7$\mu$m (Two columns connected in series with column coupler) column was used. An Acquity UPLC Peptide CSH C18 Vanguard Pre-Column 130Å 5 x 2.1mm, 1.7$\mu$m pre-column was used. Mobile phase A (Buffer solution 4mM: Methanol: TFA (950:50:1, v/v/v%) adjusted to pH 7.00 with Ammonia solution) and Mobile phase B (Acetonitrile: Methanol: Water: TFA (500:450:50:1, v/v/v/v%)) were used at a ratio 26:74. As a diluent, 0,025% v/v Ammonia in water was used. The mobile phases were filtered through a 0.45 $\mu$m membrane filter and sonicated before use. It was pumped through the column at a flow rate of 0.07 ml/min. Injection volume was 4 $\mu$l and the column was maintained at 25°C. The detection was monitored at 215 nm and the run time was set as 145 minutes. The percentage of other impurities are calculated by the following equation:

$$\% \text{ Impurity} = (As \times Wst \times P \times fds \times Ds \times Cf)/(Ast \times Ws \times fdst \times C)$$

Where,

As: Impurity peak area in test solution
Ast: Liraglutide peak area in reference solution
Wst: Weight of Liraglutide standard in Reference Solution (mg)
Ws: Weight of sample in test solution (g)
P: Purity of Liraglutide standard as is (%), into account KF determination before use (H)

$$P = P \text{ Liraglutide} \times (100 - (H))/100$$

fdst: Dilution factor of Reference Solution (2500)
fdS: Dilution factor of test solution (10)
Ds: Density of product (g/mL)
C: Content of Liraglutide in 1 mL, in mg (6.0)
Cf: Correction factor (1 / RRF = Liraglutide RF /Impurity RF)

The term "unknown impurity" as used herein refers to an impurity of unknown structure having a specific relative retention time (RRT or $t_{Rr}$) in each case. The percentage of each impurity is calculated as explained above from the results of the analysis under the UPLC conditions set forth above.

The term "analogue" as used herein referring to a peptide means a modified peptide wherein one or more amino acid residues of the peptide have been substituted by other amino acid residues and/or wherein one or more amino acid residues have been deleted from the peptide and/or wherein one or more amino acid residues have been deleted from the peptide and or wherein one or more amino acid residues have been added to the peptide. Such addition or deletion of amino acid residues can take place at the N-terminal of the peptide and/or at the C-terminal of the peptide.

The term "derivative" as used herein in relation to a parent peptide means a chemically modified parent protein or an analogue thereof, wherein at least one substituent is not present in the parent protein or an analogue thereof, i.e. a parent protein which has been covalently modified. Typical modifications are amides, carbohydrates, alkyl groups, acyl groups, esters, PEGylations and the like.

The term "GLP-1 agonist" or "GLP-1 analogues", as used herein refer to a class of active ingredients that reduce blood sugar and energy intake by activating the GLP-1 receptor. They mimic the actions of the endogenous incretin hormone GLP-1 that is released by the gut after eating. Examples of GLP-1 agonists known in the art are dulaglutide, albiglutide, liraglutide, semaglutide, exenatide, lixisenatide and tirzepatide. It will be appreciated that a GLP-1 agonist may exist as a pharmaceutically acceptable salt, where one or more ionizable functional groups of its amino acid residues associate with a counterion.

All percentages, parts and ratios herein used are by weight unless specifically noted otherwise. As used herein, the term "about" refers to a range that is ±10%, preferably ±5%, or more preferably ±1% of a value with which the term is associated.

Unless otherwise indicated, all the analytical methods are carried out according to the European Pharmacopoeia 10th edition.

The inventors have found out that a pharmaceutical composition prepared according to the method of the invention has a lower level of impurities and lower quantity of fibrils over time than when prepared by other methods involving heat treatment. The heat treatment of the fibrils is in principle carried out with the aim of dissolving the fibrils formed and avoiding its appearance. Surprisingly, the tendency to form fibrils is reduced in a method according to the invention, which employs low temperatures in the preparation of the pharmaceutical compositions.

The first aspect of the invention relates to a method for the preparation of a pharmaceutical composition comprising a GLP-1 agonist, a tonicity modifier, a buffering agent and a preservative which comprises the following steps:

  a. preparing a final solution comprising the tonicity modifier, the buffering agent, the preservative, the GLP-1 agonist and water for injection; and
  b. adjusting the pH of the final solution between 6.5-8.5, preferably between 7.5-8.5 with a pH adjuster;

wherein the final solution is prepared at a temperature between 2-15°C, preferably 2-10°C, more preferably 2-8 °C, most preferably 3-6°C.

In a preferred embodiment, the final solution may be prepared by mixing a first solution comprising the tonicity modifier, the buffering agent, the preservative, and water for injection; and a second solution comprising a GLP-1 agonist and water for injection, wherein the first and second solutions are prepared at a temperature between 2-15°C, preferably 2-10°C, more preferably 2-8 °C, most preferably 3-6°C.

In another preferred embodiment, only one final solution is prepared by mixing the tonicity modifier, the buffering agent, the preservative, the GLP-1 agonist and water for injection at a temperature between 2-15°C, preferably 2-10°C, more preferably 2-8 °C, most preferably 3-6°C.

In an embodiment, the method may further comprise bubbling nitrogen gas in any of the first, second and/or final solutions, preferably until the dissolved oxygen content is < 2.0 ppm. It has been found out that bubbling nitrogen gas helps to avoid the formation of fibrils, particularly when the dissolved oxygen content is kept below 2.0 ppm.

In an embodiment, the method may further comprise filtering the final solution through a 0.2 $\mu$m (e.g. 0.22 $\mu$m) pore size filter. Filtering the final solution through a 0.2 $\mu$m pore size filter delays the formation of fibrils.

In an embodiment, the GLP-1 agonist may be selected from the group consisting of dulaglutide, albiglutide, liraglutide, semaglutide, exenatide, lixisenatide, tirzepatide or mixtures thereof, preferably liraglutide, semaglutide or tirzepatide, more preferably liraglutide.

In an embodiment, the tonicity modifier may be selected from the group consisting of mannitol, sorbitol, lactose, propylene glycol dextrose, trehalose, sodium chloride, potassium chloride, glycerol, glycerin or mixtures thereof, preferably the tonicity modifier is propylene glycol.

In an embodiment, the buffering agent may be selected from the group consisting of sodium dihydrogen phosphate, dibasic sodium phosphate, phosphoric acid, sodium phosphate, acetic acid, sodium acetate, carbonic acid, sodium bicarbonate, sodium carbonate, citric acid, citrate, meglumine, glycine, histidine, lysine, arginine, asparagine, glutamic acid, sodium glutamate, tris (hydroxymethyl)-aminomethane, methionine, Hepes, maleic acid, malic acid, lactate or mixtures thereof, preferably the buffering agent is dibasic sodium phosphate.

In an embodiment, the preservative may be selected from the group consisting of phenol, m-cresol, methyl p-hydroxybenzoate, propyl p-hydroxybenzoate, benzoic acid, benzyl alcohol, benzyl benzoate, 2-phenoxyethanol, butyl p-hydroxybenzoate, 2-phenylethanol, benzyl alcohol, chlorobutanol, acetone sodium bisulfite, benzalkonium chloride, benzethonium chloride and thiomerosal, or any combinations thereof, preferably preservative is phenol.

In an embodiment, the method further comprises filling aseptically the final solution into cartridges flushed with nitrogen gas. The nitrogen gas flushed in the cartridges delays the appearance of fibrils. In an embodiment, no air bubbles remain inside the cartridges. The inventors have surprisingly found that when air bubbles remain in the cartridge, even if nitrogen is flushed, the concentration of fibrils is higher in the pharmaceutical composition according to the invention.

In a further disclosure, a pharmaceutical composition obtainable by the method according to the first aspect is disclosed.

In a further disclosure, the pharmaceutical composition has an oxygen content below 2.0 ppm.

In a further disclosure, a cartridge comprising the pharmaceutical composition according to the above disclosure is disclosed.

Further embodiments of the disclosure can be found in the following numbered clauses:

  1. A method for the preparation of a pharmaceutical composition comprising a GLP-1 agonist, a tonicity modifier, a buffering agent and a preservative which comprises the following steps:

    a. preparing a final solution comprising the tonicity modifier, the buffering agent, the preservative, the GLP-1 agonist and water for injection; and
    b. adjusting the pH of the final solution between 6.5-8.5, preferably between 7.5-8.5, with a pH adjuster;

  wherein the final solution is prepared at a temperature between 2-15°C, preferably 2-10°C, more preferably 2-8 °C, most preferably 3-6°C.

2. The method according to clause 1, wherein the final solution is prepared by mixing a first solution comprising the tonicity modifier, the buffering agent, the preservative, and water for injection; and a second solution comprising a GLP-1 agonist and water for injection, wherein the first and second solutions are prepared at a temperature between 2-15°C, preferably 2-10°C, more preferably 2-8 °C, most preferably 3-6°C.

3. The method according to any of the preceding clauses, comprising bubbling nitrogen gas in any of the first, second and/or final solution, preferably until the dissolved oxygen content is < 2.0 ppm.

4. The method according to any of the preceding clauses, comprising filtering the final solution through a 0.2 $\mu$m pore size filter.

5. The method according to any of the preceding clauses, wherein the GLP-1 agonist is selected from the group consisting of dulaglutide, albiglutide, liraglutide, semaglutide, exenatide, lixisenatide, tirzepatide or mixtures thereof, preferably liraglutide, semaglutide or tirzepatide, more preferably liraglutide.

6. The method according to any of the preceding clauses, wherein the tonicity modifier is selected from the group consisting of mannitol, sorbitol, lactose, propylene glycol dextrose, trehalose, sodium chloride, potassium chloride, glycerol, glycerin or mixtures thereof, preferably propylene glycol.

7. The method according to any of the preceding clauses, wherein the buffering agent is selected from the group consisting of sodium dihydrogen phosphate, dibasic sodium phosphate, sodium phosphate, sodium acetate, sodium carbonate, citrate, meglumine, glycine, histidine, lysine, arginine, asparagine, glutamic acid, sodium glutamate, tris (hydroxymethyl)-aminomethane, methionine, Hepes, maleic acid, malic acid, lactate or mixtures thereof, preferably dibasic sodium phosphate.

8. The method according to any of the preceding clauses, wherein the preservative is selected from the group consisting of phenol, m-cresol, methyl p-hydroxybenzoate, propyl p-hydroxybenzoate, benzoic acid, benzyl alcohol, benzyl benzoate, 2-phenoxyethanol, butyl p-hydroxybenzoate, 2-phenylethanol, benzyl alcohol, chlorobutanol, acetone sodium bisulfite, benzalkonium chloride, benzethonium chloride and thiomerosal, or any combinations thereof, preferably phenol.

9. The method according to any of the preceding clauses, comprising filling aseptically the final solution into cartridges flushed with nitrogen gas.

10. The method according to any of the preceding clauses, wherein no air bubbles remain inside the cartridges.

17. A method according to any of the clauses 1-10, wherein the final solution temperature does not exceed 15°C.

18. A method according to any of the clauses 1-10, wherein the final solution temperature does not exceed 14°C.

19. A method according to any of the clauses 1-10, wherein the final solution temperature does not exceed 13°C.

20. A method according to any of the clauses 1-10, wherein the final solution temperature does not exceed 12°C.

21. A method according to any of the clauses 1-10, wherein the final solution temperature does not exceed 11°C.

22. A method according to any of the clauses 1-10, wherein the final solution temperature does not exceed 10°C.

23. A method according to any of the clauses 1-10, wherein the final solution temperature does not exceed 9°C.

24. A method according to any of the clauses 1-10, wherein the final solution temperature does not exceed 8°C.

EXAMPLES

Example 1. Preparation of pharmaceutical compositions comprising liraglutide.

Two pharmaceutical compositions comprising Liraglutide where prepared with the following concentrations of GLP-1 agonist, tonicity modifier, buffering agent, preservative and water for injection (WFI):

| Composition | Liraglutide | Propylene glycol | Dibasic sodium phosphate | Phenol | WFI |
|---|---|---|---|---|---|
| GLN-1 | 0,60 | 1,40 | 0,14 | 0,55 | to 100,00 |
| Comp. example 1 | 0,60 | 1,40 | 0,14 | 0,55 | to 100,00 |

The amount of the different components is expressed as percentages by weight (w/w %) of the total composition.

Both formulations were prepared through different methods. While formulation "GLN-1" was prepared according to the method described in the present invention, formulation "Comp. example 1" was prepared by the prior art method.

Formulation "GLN-1" was manufactured by preparing a first solution comprising propylene glycol, dibasic sodium phosphate, phenol and water for injection. In another container, a second solution comprising liraglutide and water for injection was prepared. Then, a final solution was prepared by mixing the said first and the said second solution. The first, second and final solutions were prepared at a temperature between 2-8 °C. Nitrogen gas was bubbled in the first, second and final solution, to maintain the dissolved oxygen content below 2.0 ppm. Then, the pH of the final solution was adjusted to 8.15 with HCl and/or NaOH. Eventually, the final solution was filtered through a 0.22 $\mu$m filter.

Formulation "Comp. example 1" was manufactured by preparing a first solution comprising propylene glycol, dibasic sodium phosphate, phenol and WFI. In another container, a second solution comprising liraglutide and WFI was prepared and heated to 60-70° C and kept for 30 minutes at this temperature. After heat treatment, the second solution was cooled to 22° C. Afterwards, the first and second solutions were mixed to prepare the final solution, and pH adjusted to 8.15 using HCl and/or NaOH. Nitrogen gas was bubbled in the first, second and final solution, to maintain the dissolved oxygen content below 2.0 ppm. Finally, the formulation was filtered through a 0.22 μm filter.

## Example 2. Impurity content

Both formulations obtained in Example 1 were analysed by UPLC as described above for impurity content at two different times, freshly prepared (t=0) and at 1 month stored at 25 °C / 60 % RH. The following results were obtained:

|  | GLN | | Comp. Ex. 1 | |
| --- | --- | --- | --- | --- |
|  | t=0 | t=1 | t=0 | t=1 |
| Unknown impurity ($t_{Rr}$ = 1.08) | 0,16 | 0,14 | ND | ND |
| Unknown impurity ($t_{Rr}$ = 1.106) | ND | ND | 0,16 | ND |
| Imp E ($t_{Rr}$ = 1.11) | 0,10 | 0,18 | 0,11 | 0,540 |
| Unknown impurity ($t_{Rr}$ = 1.14) | 0,18 | 0,04 | 0,14 | 0,750 |
| Imp D ($t_{Rr}$ = 1.17) | ND | 0,07 | ND | 0,060 |
| Unknown impurity ($t_{Rr}$ = 1.20) | 0,16 | 0,06 | 0,11 | 0,520 |
| Unknown impurity ($t_{Rr}$ = 1.22) | 0,05 | ND | 0,05 | 0,620 |
| Unknown impurity ($t_{Rr}$ = 1.23) | ND | ND | ND | 0,460 |
| Unknown impurity ($t_{Rr}$ = 1.36) | ND | 0,09 | ND | 2,950 |
| Imp F ($t_{Rr}$ = 1.41) | ND | 0,16 | ND | 0,370 |
| Imp G ($t_{Rr}$ = 1.45) | ND | 0,16 | ND | 0,350 |
| Imp I ($t_{Rr}$ = 1.50) | ND | ND | 0,04 | 0,030 |
| Imp H ($t_{Rr}$ = 1.52) | 0,11 | 0,14 | 0,35 | 0,500 |
| Total impurities (%) | 0,76 | 1,04 | 0,96 | 7,15 |

All amounts of impurities are expressed as a percentage, "ND" corresponds to not detected. As it can be seen, the amount of each of the impurities over time is lower in GLN-1, as well as the percentage of total impurities.
The chemical name of the above-mentioned identified impurities is as follows:

| Impurity name | Chemical name |
| --- | --- |
| Impurity D | Des Gly[37] Liraglutide |
| Impurity E | D His[7] Liraglutide |
| Impurity F | Des His[7] Liraglutide |
| Impurity G | Des His[7] - Ala[8] Liraglutide |
| Impurity I | Des His[7]- Gly[10] |
| Impurity H | Des His[7]- Glu[9] |

## Example 3. Thioflavin T (ThT) Fibrillation Estimation Assay

Low physical stability of a peptide may lead to amyloid fibril formation, which is observed as well-ordered, thread-like macromolecular structures in the sample eventually resulting in gel formation. This has traditionally been measured by visual inspection of the sample. However, that kind of measurement is very subjective and depends on the observer. Therefore, the application of a small molecule indicator test is much more advantageous. Thioflavin T (ThT) is such a test and has a distinct fluorescence signature when binding to fibrils.

To minimise costs related to the active ingredient and being able to estimate the fibrils in the compositions (at 6mg/mL) by ThT fibrillation assay, Bovine serum albumin (BSA) was used for the standard curve of the assay, since BSA has similar tendency to fibril formation as liraglutide. To this aim, a BSA standard curve was prepared from 0.188 to 0.003 mM and a Thioflavin-T working solution at 50 $\mu$M. After preparation of standard concentration and one blank (PBS), the BSA standard samples at 80°C for 30 min and then cool at room temperature for fibril generation.

Preparation of the samples

- Preparation of standard Blank: Add 50$\mu$L of heated and cooled PBS into 96 well plate in duplicate.
- Preparation of Standard: Add 50 $\mu$L of heated and cooled BSA (0.188 mM - 0.003 mM) each into 96 well plate in duplicate.
- Preparation of Liraglutide RLD /Test samples Blank: Add 50$\mu$L of Milli-Q water into 96 well plate in duplicate.
- Preparation of Liraglutide RLD /Test samples: Add 50 $\mu$L RLD /Test samples (50$\mu$M) into 96 well plate in duplicate. Then read the plate as per method created in Micro plate reader.

After addition of all blanks, standard curve concentrations and liraglutide (RLD)/ test sample concentrations in the plate, further 150$\mu$L of thioflavin-T working solution(50$\mu$M) were added into each well and mixed gently by pipetting. Read the plate as per the following microplate reader conditions:
(Read Mode: Fluorescence; excitation wavelength: 440nm; emission wavelength: 482nm; bandwidth excitation: 9 nm; bandwidth emission: 15 nm; plate type: 96 well standard opaque; plate height: 14.6mm; plate Shake: 60 seconds, orbital, medium (before first read); PMT and optics: 6 flashes/read; Read from: Top; Read height: 1.00mm).
The measurements were performed using a CLARIOstar® Plus fluorescence microplate reader.

Calculations and results

Plotted the standard curve with the blank subtracted values of standards on Y-axis and their concentrations on X-axis.
Derived slope (Mstd), intercept (Cstd) and regression(R2) from the straight-line equation.
The following compositions were measured according to the above ThT fibrillation assay:

| Composition | Temperature (°C) | pH |
|---|---|---|
| GLN-1 | 8 | 8.15 |
| Comp. Ex. 2 | 60 | 7 |
| Comp. Ex. 3 | 80 | 7 |
| Comp. Ex. 4 | 60 | 8.15 |

GLN-1 was prepared as described in Example 1. Comp. Ex. 2-4 were prepared as Comp. Ex. 1, but the pH and/or temperature were modified according to the above table. After preparation, the samples were stored for 1 month at 30°C / Relative Humidity 65% and analysed. Afterwards, samples were analysed according to the ThT fibrillation assay detailed above and the following results were obtained:

| Composition | Fibrils content ($\mu$M) |
|---|---|
| GLN-1 | 0,0178 |
| Comp. Ex. 2 | 0,0392 |
| Comp. Ex. 3 | 0,0518 |
| Comp. Ex. 4 | 0,1217 |

**Claims**

1. A method for the preparation of a pharmaceutical composition comprising a GLP-1 agonist, a tonicity modifier, a buffering agent and a preservative which comprises the following steps:

   a. preparing a final solution comprising the tonicity modifier, the buffering agent, the preservative, the GLP-1 agonist and water for injection; and
   b. adjusting the pH of the final solution between 6.5-8.5, preferably between 7.5-8.5, with a pH adjuster;

   wherein the final solution is prepared at a temperature between 2-15°C, preferably 2-10°C, more preferably 2-8 °C, most preferably 3-6°C.

2. The method according to claim 1, wherein the final solution is prepared by mixing a first solution comprising the tonicity modifier, the buffering agent, the preservative, and water for injection; and a second solution comprising a GLP-1 agonist and water for injection, wherein the first and second solutions are prepared at a temperature between 2-15°C, preferably 2-10°C, more preferably 2-8 °C, most preferably 3-6°C.

3. The method according to any of the preceding claims, comprising bubbling nitrogen gas in any of the first, second and/or final solution, preferably until the dissolved oxygen content is < 2.0 ppm.

4. The method according to any of the preceding claims, comprising filtering the final solution through a 0.2 $\mu$m pore size filter.

5. The method according to any of the preceding claims, wherein the GLP-1 agonist is selected from the group consisting of dulaglutide, albiglutide, liraglutide, semaglutide, exenatide, lixisenatide, tirzepatide or mixtures thereof, preferably liraglutide, semaglutide or tirzepatide, more preferably liraglutide.

6. The method according to any of the preceding claims, wherein the tonicity modifier is selected from the group consisting of mannitol, sorbitol, lactose, propylene glycol dextrose, trehalose, sodium chloride, potassium chloride, glycerol, glycerin or mixtures thereof, preferably propylene glycol.

7. The method according to any of the preceding claims, wherein the buffering agent is selected from the group consisting of sodium dihydrogen phosphate, dibasic sodium phosphate, sodium phosphate, sodium acetate, sodium carbonate, citrate, meglumine, glycine, histidine, lysine, arginine, asparagine, glutamic acid, sodium glutamate, tris (hydroxymethyl)-aminomethane, methionine, Hepes, maleic acid, malic acid, lactate or mixtures thereof, preferably dibasic sodium phosphate.

8. The method according to any of the preceding claims, wherein the preservative is selected from the group consisting of phenol, m-cresol, methyl p-hydroxybenzoate, propyl p-hydroxybenzoate, benzoic acid, benzyl alcohol, benzyl benzoate, 2-phenoxyethanol, butyl p-hydroxybenzoate, 2-phenylethanol, benzyl alcohol, chlorobutanol, acetone sodium bisulfite, benzalkonium chloride, benzethonium chloride and thiomerosal, or any combinations thereof, preferably phenol.

9. The method according to any of the preceding claims, comprising filling aseptically the final solution into cartridges flushed with nitrogen gas.

10. The method according to any of the preceding claims, wherein no air bubbles remain inside the cartridges.

**Patentansprüche**

1. Verfahren zur Herstellung einer pharmazeutischen Zusammensetzung, umfassend einen GLP-1-Agonisten, ein Tonizitätsmittel, einen Puffer und ein Konservierungsmittel, welches die folgenden Schritte umfasst:

   a. Herstellen einer Endlösung, umfassend das Tonizitätsmittel, den Puffer, das Konservierungsmittel, den GLP-1-Agonisten und Wasser für Injektionszwecke; und
   b. Einstellen des pH-Werts der Endlösung auf einen Wert zwischen 6,5-8,5, vorzugsweise zwischen 7,5-8,5, mit einem pH-Regulator;

wobei die Endlösung bei einer Temperatur zwischen 2-15 °C, vorzugsweise 2-10 °C, bevorzugter 2-8 °C, am bevorzugtesten 3-6 °C hergestellt wird.

2. Verfahren nach Anspruch 1, wobei die Endlösung durch Mischen einer ersten Lösung, umfassend das Tonizitätsmittel, den Puffer, das Konservierungsmittel und Wasser für Injektionszwecke, und einer zweiten Lösung, umfassend einen GLP-1-Agonisten und Wasser für Injektionszwecke, hergestellt wird, wobei die erste und die zweite Lösung bei einer Temperatur zwischen 2-15 °C, vorzugsweise 2-10 °C, bevorzugter 2-8 °C, am bevorzugtesten 3-6 °C hergestellt werden.

3. Verfahren nach einem der vorhergehenden Ansprüche, umfassend das Durchleiten (Bubbling) von Stickstoffgas durch die erste, die zweite und/oder die Endlösung, vorzugsweise bis der Gehalt an gelöstem Sauerstoff < 2,0 ppm beträgt.

4. Verfahren nach einem der vorhergehenden Ansprüche, umfassend das Filtern der Endlösung durch einen Filter mit einer Porengröße von 0,2 $\mu$m.

5. Verfahren nach einem der vorhergehenden Ansprüche, wobei der GLP-1-Agonist ausgewählt ist aus der Gruppe bestehend aus Dulaglutid, Albiglutid, Liraglutid, Semaglutid, Exenatid, Lixisenatid, Tirzepatid oder Mischungen davon, vorzugsweise Liraglutid, Semaglutid oder Tirzepatid, bevorzugter Liraglutid.

6. Verfahren nach einem der vorhergehenden Ansprüche, wobei das Tonizitätsmittel ausgewählt ist aus der Gruppe bestehend aus Mannit, Sorbit, Lactose, Propylenglykol, Dextrose, Trehalose, Natriumchlorid, Kaliumchlorid, Glycerol, Glycerin oder Mischungen davon, vorzugsweise Propylenglykol.

7. Verfahren nach einem der vorhergehenden Ansprüche, wobei der Puffer ausgewählt ist aus der Gruppe bestehend aus Natriumdihydrogenphosphat, Dinatriumhydrogenphosphat, Natriumphosphat, Natriumacetat, Natriumcarbonat, Citrat, Meglumin, Glycin, Histidin, Lysin, Arginin, Asparagin, Glutaminsäure, Natriumglutamat, Tris(hydroxymethyl)aminomethan, Methionin, HEPES, Maleinsäure, Äpfelsäure, Lactat oder Mischungen davon, vorzugsweise Dinatriumhydrogenphosphat.

8. Verfahren nach einem der vorhergehenden Ansprüche, wobei das Konservierungsmittel ausgewählt ist aus der Gruppe bestehend aus Phenol, m-Kresol, Methyl-p-hydroxybenzoat, Propyl-p-hydroxybenzoat, Benzoesäure, Benzylalkohol, Benzylbenzoat, 2-Phenoxyethanol, Butyl-p-hydroxybenzoat, 2-Phenylethanol, Chlorbutanol, Aceton-Natriumbisulfit, Benzalkoniumchlorid, Benzethoniumchlorid und Thiomersal oder beliebigen Kombinationen davon, vorzugsweise Phenol.

9. Verfahren nach einem der vorhergehenden Ansprüche, umfassend das aseptische Abfüllen der Endlösung in mit Stickstoffgas gespülte Karpullen (Cartridges).

10. Verfahren nach einem der vorhergehenden Ansprüche, wobei keine Luftblasen in den Karpullen verbleiben.

**Revendications**

1. Procédé de préparation d'une composition pharmaceutique comprenant un agoniste du GLP-1, un agent de tonicité, un agent tampon et un conservateur, lequel comprend les étapes suivantes :

   a. la préparation d'une solution finale comprenant l'agent de tonicité, l'agent tampon, le conservateur, l'agoniste du GLP-1 et de l'eau pour préparations injectables ; et
   b. l'ajustement du pH de la solution finale entre 6,5 et 8,5, de préférence entre 7,5 et 8,5, avec un agent d'ajustement du pH ;

   dans lequel la solution finale est préparée à une température comprise entre 2 et 15 °C, de préférence entre 2 et 10 °C, plus préférablement entre 2 et 8 °C, le plus préférablement entre 3 et 6 °C.

2. Procédé selon la revendication 1, dans lequel la solution finale est préparée en mélangeant une première solution comprenant l'agent de tonicité, l'agent tampon, le conservateur et de l'eau pour préparations injectables ; et une seconde solution comprenant un agoniste du GLP-1 et de l'eau pour préparations injectables, dans lequel les première et seconde solutions sont préparées à une température comprise entre 2 et 15 °C, de préférence entre 2 et 10 °C, plus préférablement entre 2 et 8 °C, le plus préférablement entre 3 et 6 °C.

3. Procédé selon l'une quelconque des revendications précédentes, comprenant le barbotage d'azote gazeux dans l'une quelconque des première, seconde et/ou solution finale, de préférence jusqu'à ce que la teneur en oxygène dissous soit < 2,0 ppm.

4. Procédé selon l'une quelconque des revendications précédentes, comprenant la filtration de la solution finale à travers un filtre ayant une taille de pores de 0,2 μm.

5. Procédé selon l'une quelconque des revendications précédentes, dans lequel l'agoniste du GLP-1 est choisi dans le groupe constitué par le dulaglutide, l'albiglutide, le liraglutide, le sémaglutide, l'exénatide, le lixisénatide, le tirzépatide ou leurs mélanges, de préférence le liraglutide, le sémaglutide ou le tirzépatide, plus préférablement le liraglutide.

6. Procédé selon l'une quelconque des revendications précédentes, dans lequel l'agent de tonicité est choisi dans le groupe constitué par le mannitol, le sorbitol, le lactose, le propylène glycol, le dextrose, le tréhalose, le chlorure de sodium, le chlorure de potassium, le glycérol, la glycérine ou leurs mélanges, de préférence le propylène glycol.

7. Procédé selon l'une quelconque des revendications précédentes, dans lequel l'agent tampon est choisi dans le groupe constitué par le dihydrogénophosphate de sodium, le phosphate de sodium dibasique, le phosphate de sodium, l'acétate de sodium, le carbonate de sodium, le citrate, la méglumine, la glycine, l'histidine, la lysine, l'arginine, l'asparagine, l'acide glutamique, le glutamate de sodium, le tris(hydroxyméthyl)-aminométhane, la méthionine, l'Hepes, l'acide maléique, l'acide malique, le lactate ou leurs mélanges, de préférence le phosphate de sodium dibasique.

8. Procédé selon l'une quelconque des revendications précédentes, dans lequel le conservateur est choisi dans le groupe constitué par le phénol, le m-crésol, le p-hydroxybenzoate de méthyle, le p-hydroxybenzoate de propyle, l'acide benzoïque, l'alcool benzylique, le benzoate de benzyle, le 2-phénoxyéthanol, le p-hydroxybenzoate de butyle, le 2-phényléthanol, l'alcool benzylique, le chlorobutanol, le bisulfite de sodium et d'acétone, le chlorure de benzalkonium, le chlorure de benzéthonium et le thiomersal, ou l'une quelconque de leurs combinaisons, de préférence le phénol.

9. Procédé selon l'une quelconque des revendications précédentes, comprenant le remplissage aseptique de la solution finale dans des cartouches balayées à l'azote gazeux.

10. Procédé selon l'une quelconque des revendications précédentes, dans lequel aucune bulle d'air ne subsiste à l'intérieur des cartouches.

**EP 4 656 182 B1**

**REFERENCES CITED IN THE DESCRIPTION**

**Patent documents cited in the description**

- US 2023159606 A1
- WO 2006051110 A2
- US 8748376 B2
- WO 2020127476 A1
- WO 2021123228 A1